# EUROPÄISCHE PATENTSCHRIFT

(11) **EP 0 596 436 B1**
(45) Veröffentlichungstag und Bekanntmachung des Hinweises auf die Patenterteilung: **04.02.1998**
(21) Anmeldenummer: 93117663.0
(22) Anmeldetag: 02.11.1993
(51) Int. Cl.: A61B 17/39

(54) **Elektrochirurgisches Instrument**
Electrosurgical instrument
Appareil électro-chirurgical

(30) Priorität: 06.11.1992 DE 4237518
(43) Veröffentlichungstag der Anmeldung: 11.05.1994
(73) Patentinhaber: HÜTTINGER MEDIZINTECHNIK GmbH & CO. KG, D-79224 Umkirch (DE)
(72) Erfinder: Bissinger, Günther, D-79331 Teningen (DE); Dold, Max, D-79227 Schallstadt-Wolfenweiler (DE); Scholz, Clemens, Dr.-Ing., D-79106 Freiburg (DE)
(74) Vertreter: Wolf, Eckhard, Dr.-Ing.

(56) Entgegenhaltungen:
- EP-A- 0 484 671
- DE-A- 2 442 605
- DE-A- 2 734 847
- DE-U- 9 209 665
- FR-A- 2 310 137

## Beschreibung

Die Erfindung betrifft ein elektrochirurgisches Instrument zur bipolaren Koagulation oder Gewebstrennung mit einem eine Axialbohrung aufweisenden Gehäuse, einem in die Axialbohrung des Gehäuses mit seinem proximalen Ende eingreifenden und in dieser längsverschiebbar angeordneten Führungsrohr, einem durch das Führungsrohr axial verschiebbar hindurchgreifenden, mit seinem proximalen Ende durch das Führungsrohr hindurch in die Axialbohrung des Gehäuses eingreifenden und in diesem axial arretierbaren Elektrodenrohr und mit mindestens einem sich durch das Innere des Elektrodenrohrs erstreckenden, und gegenüber diesem elektrisch isolierten Drahtstück, wobei am distalen Ende zwei über das Elektrodenrohr und das Führungsrohr überstehende, beim Verschieben des Führungsrohrs radial zangenartig gegeneinander bewegbare, an ihren freien Enden metallische Elektroden tragende, gegeneinander isolierte Branchen und am proximalen Ende zwei mit den Elektroden elektrisch verbundene, mit Hochfrequenzstrom beaufschlagbare Kontaktelemente angeordnet sind, und wobei das Gehäuse am einen Betätigungsschenkel und das Führungsrohr am anderen Betätigungsschenkel einer vorzugsweise als Feder- oder Scherengriff ausgebildeten, zweischenkeligen Betätigungsvorrichtung angelenkt sind.

Bipolare Koagulationszangen, wie sie beispielsweise aus der DE-A-27 34 847 bekannt sind, weisen zwei federnd ausgebildete, elektrisch voneinander isolierte Branchen am distalen Ende auf, die beim Verschieben des Führungsrohrs mit Hilfe eines geeigneten Griffes zangenartig gegeneinander bewegt werden können. Dementsprechend muß der Operateur die zunächst auseinandergespreizten Branchen in eine solche Lage bringen, daß beim Verschieben des Führungsrohrs und dem dadurch bewirkten Zusammenführen der Branchen das zu koagulierende oder trennende Gewebe zwischen die Branchen eingeklemmt wird. Durch Anlegen einer Hochfrequenzspannung an den proximalen Kontaktelementen fließt ein Hochfrequenzstrom über das eingeklemmte Gewebe, das bei ausreichend hohem Stromfluß erwärmt und koaguliert wird. Bei dem bekannten Koagulationsinstrument ist das Elektrodenrohr mit Hilfe einer Stellmutter am Gehäuse in beliebiger Drehorientierung um seine Längsachse lösbar befestigt. Die Stellmutter muß daher sowohl beim Auswechseln des Elektrodenrohrs als auch beim Verstellen der Winkellage gelöst und anschließend wieder angezogen werden. Dabei wird das Elektrodenrohr kraftschlüssig zwischen zwei klemmbacken eingeklemmt. Aufgrund dieser rein kraftschlüssigen Verbindung ist nicht sichergestellt, daß das Elektrodenrohr beim Anklemmen bezüglich der Kontaktelemente axial richtig positioniert wird. Abgesehen davon ist damit eine einhändige Veränderung der Winkellage des Elektrodenrohrs beispielsweise während der Operation nicht möglich.

Ausgehend hiervon liegt der Erfindung die Aufgabe zugrunde, ein elektrochirurgisches Instrument der eingangs angegebenen Art zu entwickeln, bei welchem das Elektrodenrohr mit seinen Branchen mit wenigen Handgriffen rasch und zuverlässig ausgetauscht und auch während der Operation einhändig in seiner Drehlagenorientierung verändert werden kann.

Zur Lösung dieser Aufgabe wird die im Patentanspruch 1 angegebene Merkmalskombination vorgeschlagen. Weitere vorteilhafte Ausgestaltungen und Weiterbildungen der Erfindung ergeben sich aus den abhängigen Ansprüchen.

Die erfindungsgemäße Lösung geht von dem Gedanken aus, daß zum raschen und zuverlässigen Austausch des Elektrodenrohrs eine drehorientierungsunabhängige lösbare axiale Rastverbindung besonders geeignet ist, vorausgesetzt, daß eine nachträgliche Änderung der Drehorientierung besonders einfach, möglichst einhändig möglich ist. Um dies zu erreichen, sind jedoch zusätzliche Maßnahmen erforderlich, die ein Verdrehen des Instrumentengehäuses gegenüber der Betätigungsvorrichtung sowie eine Drehkupplung zwischen Instrumentengehäuse und Elektrodenrohr gewährleisten. Dementsprechend wird gemäß der Erfindung vorgeschlagen, daß das Instrumentengehäuse um die Achse seiner Axialbohrung am einen Schenkel der Betätigungsvorrichtung drehbar gelagert ist, daß das Elektrodenrohr mit beliebiger Drehorientierung um seine Längsachse am Instrumentengehäuse axial einrastbar und mit einer am Gehäuse dreh- und verschiebefest verankerbaren, vorzugsweise einen Stromanschluß für die Kontaktelemente enthaltenden Kupplungsvorrichtung drehfest verbindbar ist.

Eine erste vorteilhafte Ausführungsform der Erfindung sieht vor, daß das Elektrodenrohr als Schutzrohr ausgebildet ist und daß in dem Elektrodenrohr zwei gegeneinander elektrisch isolierte, an ihren stirnseitigen Enden eine der Branchen tragende Drahtstücke angeordnet sind. Alternativ dazu ist es möglich, daß das Elektrodenrohr als Außenleiter und das Drahtstück als Innenleiter eines Koaxialleiters ausgebildet ist, wobei eine der Branchen am Elektrodenrohr und die andere Branche am Drahtstück angeordnet ist. Das metallische Elektrodenrohr trägt vor allem bei der letztgenannten Ausführungsform zweckmäßig eine isolierende Schutzhülle.

Zur Herstellung der drehfesten Verbindung wird zweckmäßig eine Bajonettverbindung zwischen der Kupplungsvorrichtung und dem Instrumentengehäuse verwendet, bei der die Kupplungsvorrichtung mit einer auf einen radial überstehenden Bajonettzapfen des Gehäuses aufsteckbaren Bajonettkulisse versehen sein kann. Die Kupplungsvorrichtung kann dabei zusätzlich mit einer Buchse zur Aufnahme der Kontaktelemente des Elektrodenrohrs versehen sein. Die Kontaktelemente können beispielsweise als achsparallele, über das proximale Elektrodenrohrende überstehende Steckstifte ausgebildet sein, während die Kupplungsvorrichtung zwei achsparallele Steckhülsen zur Aufnahme der Steckstifte aufweisen kann. Besonders einfach handhabbar ist jedoch eine Anordnung, bei der die Kontaktelemente gegeneinander isoliert in axialem Abstand voneinander in der Nähe des proximalen Elektrodenrohrendes angeordnet sind und eine umlaufende, vorzugsweise zylindrische Kontaktfläche aufweisen, wobei das eine Kontaktelement ein das proximale Elektrodenrohrende begrenzendes, vorzugsweise seitlich abgeflachtes Formstück aufweisen kann, das mit einem komplementären Formstück des Kupplungselements formschlüssig kuppelbar ist. In konstruktiver Hinsicht kann dies mit einer über das proximale Elektrodenrohrende koaxial überstehenden, vorzugsweise in dieses eingeschraubten, als Isolator ausgebildeten Rohrverlängerung gelöst werden, die einen an das Elektrodenrohr anschließenden Bund etwa gleichen Durchmessers wie das Elektrodenrohr und ein axial an den Bund anschließendes Rohrteil kleineren Durchmessers aufweist, wobei auf das Rohrteil ein als erstes Kontaktelement ausgebildeter Metallring und ein Isolatorring größeren Durchmessers aufsteckbar sind und an dessen freiem Ende ein den Metallring und den Isolatorring an der Rohrverlängerung festlegendes, das zweite Kontaktelement bildendes metallisches Abschlußteil mit umlaufender Kontaktfläche und rückwärtig überstehendem Formstück befestigbar ist, und wobei die durch das Elektrodenrohr hindurchgeführten starren Drahtstücke mit je einem der Kontaktelemente verbunden, vorzugsweise verschweißt oder verlötet sind.

Zur Kontaktierung zwischen Drahtstück und Metallring können der Metallring und das Verlängerungsrohr miteinander fluchtende Radialbohrungen zur Aufnahme des radial abgebogenen Endes des einen Drahtstückes aufweisen, während das andere Drahtstück mit seinem freien Ende in eine Axialbohrung des metallischen Abschlußteils eingeführt werden kann. Die Drahtstücke werden an ihren Enden vorzugsweise durch Laserschweißung mit dem zugehörigen Kontaktelement verbunden, wodurch man gleichzeitig eine Abdichtung der betreffenden Bohrungen erhält.

Vorteilhafterweise weisen die umlaufenden Kontaktflächen der Kontaktelemente und/oder die Umfangsflächen des Rohrverlängerungsbundes und des Isolatorrings im wesentlichen den gleichen Durchmesser wie das Elektrodenrohr auf. Um die drehorientierungsfreie Rastverbindung zu ermöglichen, ist das Elektrodenrohr zweckmäßig in der Nähe seines proximalen Endes mit einer zum distalen Ende weisenden umlaufenden koaxialen Rastschulter versehen, die durch eine vorzugsweise konische Eindrehung in der Elektrodenrohroberfläche gebildet sein kann. Um dazuhin das Elektrodenrohr mit wenigen einfachen Handgriffen demontieren zu können, kann das Elektrodenrohr zusätzlich mit einer zum proximalen Ende weisenden Stützschulter versehen werden, gegen die ein vorzugsweise im Gehäuse begrenzt verschiebbar angeordnetes, unter der Einwirkung einer Feder stehendes Auswurforgan andrückbar ist. Zum Lösen der Rastverbindung kann im Gehäuse ein vorzugsweise in einer radialen Gehäusebohrung entgegen der Kraft einer beispielsweise als Blattfeder ausgebildeten Rastfeder radial verschiebbarer, eine Durchstecköffnung für das Elektrodenrohr aufweisender Rastschieber vorgesehen werden, der unter der Einwirkung der Rastfeder im Bereich der Durchstecköffnung hinter die Rastschulter und/oder in die Eindrehung des Elektrodenrohrs lösbar einrastbar ist. Die Durchstecköffnung kann hierzu eine zum rückwärtigen Ende konvergierende konische Innenwand enthalten, die gegenüber der konischen Eindrehung des Elektrodenrohrs Übermaß aufweist.

Zur Verbesserung der Handhabung des Instruments bei der Winkelausrichtung wird vorgeschlagen, daß das Gehäuse in Drehrichtung vorzugsweise in Winkelabständen von jeweils 90° an der Betätigungsvorrichtung lösbar einrastbar ist. Zweckmäßig ist dazu an dem Gehäuse vorzugsweise im Bereich zwischen den beiden Betätigungsschenkeln ein beispielsweise als Wellenrad ausgebildetes Betätigungsrad radial überstehend angeordnet.

Zur Verbesserung des Gleitverhaltens beim Verschieben des Führungsrohrs im Gehäuse ist das Führungsrohr vorteilhafterweise mit einem endseitig aufgesetzten Führungs- und Anschlagring aus Kunststoff in der Axialbohrung des Gehäuses begrenzt axial geführt. Zusätzlich kann zu diesem Zweck die Axialbohrung durch eine vorzugsweise stirnseitig eingeschraubte, als stirnseitiger Anschlag ausgebildete Stopfbuchse aus Kunststoff für den Durchtritt des vorzugsweise metallischen Führungsrohrs begrenzt werden.

Eine weitere vorteilhafte Ausgestaltung der Erfindung sieht vor, daß die Kupplungsvorrichtung ein vorzugsweise mit einer Bajonettkulisse zur Verankerung am Gehäuse versehenes Hülsenteil und ein drehfest gegenüber dem Hülsenteil axial entgegen der Kraft einer Feder verschiebbares Buchsenteil aufweist, wozu das Hülsenteil mit einem in eine achsparallele Außennut des Buchsenteils eingreifenden, vorzugsweise als Schraubbolzen ausgebildeten Gleitstein versehen sein kann. Im Buchsenteil sind zweckmäßig zwei gegeneinander isoliert im Abstand voneinander angeordnete, miteinander fluchtende Buchsenringe drehfest angeordnet, von denen der eine, rückwärtig angeordnete Buchsenring eine zum proximalen Formstück des Elektrodenrohrs komplementäre Aussparung aufweisen kann. Mit dieser formschlüssigen Steckverbindung wird erreicht, daß das Elektrodenrohr um seine Achse von außen her intraoperativ gedreht werden kann. Die Drehung erfolgt über das drehfest mit dem Gehäuse verbundene Drehrad. Das Gehäuse ist über die Bajonettverbindung drehfest mit der Kupplungsvorrichtung verbunden. Innerhalb der Kupplungsvorrichtung stellt der in der Längsnut verschiebbare Gleitstein eine Drehsicherung mit der Buchsenhülse her, während über die Steckhülsen bzw. die Kontaktbuchse des Buchsenteils ein Formschluß mit den Steckkontakten des Elektrodenrohrs hergestellt wird.

Eine weitere Besonderheit der Erfindung ist darin zu sehen, daß die Elektroden eine ballige, vorzugsweise halbkugelförmige oder halbzylindrische Gestalt aufweisen, wobei die einander zugewandten Elektrodenflächen eben sind. Die Stirnflächen können entweder eben oder ebenfalls ballig sein, wodurch sich eine in axialer Richtung wirksame Querschnittsfläche ergibt, die eine mechanische Bearbeitung, z.B. eine Kompression des Gewebes, und eine Applikation des Stromes bei zum Gewebe senkrechter Instrumentenlage unter definierten Flächenverhältnissen der Elektroden ermöglicht.

Eine weitere wichtige Elektrodenform für bipolare Koagulationszangen erhält man, wenn die Elektroden zueinander parallele hakenförmige Abbiegungen aufweisen. Damit kann das Gewebe zunächst auf die konkave Seite der Haken aufgeladen und mit diesen abgehoben werden, um sie anschließend zu koagulieren oder zu präparieren. Mit der konvexen Seite der hakenförmigen Elektroden kann kurvenartig entlang eines Gewebeteils verfahren und dabei koaguliert oder verdampft werden. Dabei kann im Verlauf dieses Vorgangs der Abstand zwischen den beiden Elektroden bedarfsgerecht variiert werden. Durch Öffnen und Schließen kann mit den hakenförmigen Elektroden wahlweise mit oder ohne Strom stumpf präpariert werden.

Im folgenden wird die Erfindung anhand einiger in der Zeichnung in schematischer Weise dargestellter Ausführungsbeispiele näher erläutert. Es zeigen
- Fig. 1a und b: eine Seitenansicht und eine Draufsicht eines bipolaren Koagulationsinstruments;
- Fig. 2: einen vergrößerten Ausschnitt aus einem Koagulationsinstrument ohne Auswurfmechanismus in teilweise geschnittener Seitenansicht;
- Fig. 3: einen vergrößerten Ausschnitt aus einem Koagulationsinstrument mit Auswurfmechanismus in teilweise geschnittener Seitenansicht;
- Fig. 4a bis c: eine Seitenansicht, einen Längsschnitt und eine Rückseitenansicht einer Kupplungsvorrichtung für Steckstifte mit Kabeladapter;
- Fig. 5a bis c: eine Seitenansicht, einen Längsschnitt und eine Stirnseitenansicht einer Kupplungsvorrichtung für Segmentstecker mit Kabeladapter;
- Fig. 6a bis c: die Kupplungsvorrichtung nach Fig. 5 mit unmittelbarem Kabelanschluß;
- Fig. 7a und b: eine Seitenansicht und eine Draufsicht des proximalen Endes eines Elektrodenrohrs mit Segmentstecker;
- Fig. 7c: einen Längsschnitt durch das proximale Elektrodenrohrende nach Fig. 7a;
- Fig. 7d: eine Explosionsdarstellung des proximalen Elektrodenrohrendes;
- Fig. 7e: eine gegenüber Fig. 7a 90° um die Längsachse gedrehte Seitenansicht des proximalen Elektrodenrohrendes;
- Fig. 7f: zwei Drahtstückenden;
- Fig. 8a und b: eine Seitenansicht und eine Stirnseitenansicht des proximalen Endes eines gegenüber Fig. 7a und b abgewandelten Elektrodenrohrs mit Auswurfschulter;
- Fig. 9a und b: eine Seitenansicht und eine Stirnseitenansicht des proximalen Endes eines Elektrodenrohrs mit Steckstiften;
- Fig. 10a und b: eine Seitenansicht und eine Draufsicht des distalen Endes eines Elektrodenrohrs mit auseinandergespreizten Branchen und halbkugelförmigen Elektroden;
- Fig. 11a und b: eine Seitenansicht und eine Draufsicht des distalen Endes eines Elektrodenrohrs mit auseinandergespreizten Branchen und hakenförmig gebogenen Elektroden.

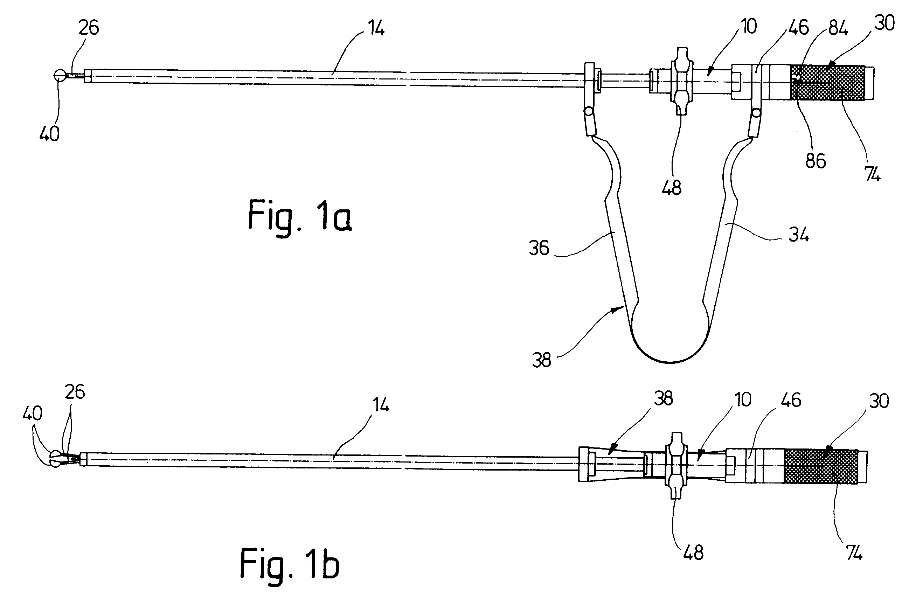

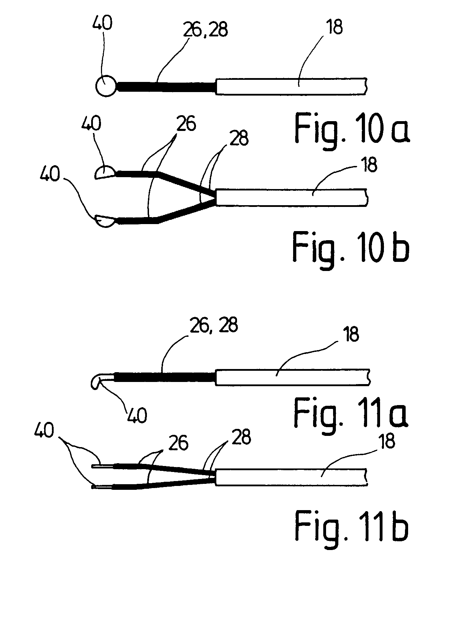

Die in der Zeichnung dargestellten elektrochirurgischen Instrumente bestehen im wesentlichen aus einem rohrförmigen Instrumentengehäuse 10, einem mit seinem proximalen Ende in eine Axialbohrung 12 des Instrumentengehäuses eingreifenden und in dieser axial verschiebbaren Führungsrohr 14, einem durch eine Axialbohrung 16 des Führungsrohrs hindurchsteckbaren, mit seinem proximalen Ende 20 innerhalb des Instrumentengehäuses 10 mit beliebiger Drehorientierung um seine Längsachse 22 einrastbaren Elektrodenrohr 18, zwei durch das Elektrodenrohr 18 hindurchgeführten, an Kontaktelementen 24 im proximalen Bereich 20 mit HF-Strom beaufschlagbaren und an ihrem distalen Ende mit zwei zangenartig elastisch aufspreizbaren Branchen 26 versehenen Drahtstücken 28 sowie einer Kupplungsvorrichtung 30 für den Anschluß an eine HF-Spannungsquelle über ein Kabel 32. Das Gehäuse 10 ist am einen Betätigungsschenkel 34 und das Führungsrohr 14 am anderen Betätigungsschenkel 36 einer Federzange 38 so angelenkt, daß beim gegenseitigen Zusammendrücken der Betätigungsschenkel 34, 36 das Führungsrohr 14 mit seinem proximalen Ende 15 in die Axialbohrung 12 hineinverschoben wird und dabei die Branchen 26 am proximalen Ende des Elektrodenrohrs 18 freigelegt werden. Da die Branchen 26 unter einer elastischen Vorspannung stehen, werden die am freien Ende der Branchen angeordneten metallischen Elektroden 40 beim Freilegen in die in Fig. 10 b und 11 b gezeigte Lage auseinandergespreizt, während sie beim Zurückverschieben des Führungsrohrs 14 durch Loslassen der Betätigungsschenkel 34, 36 zangenartig gegeneinander bewegt werden (vgl. Fig. 1 b). Zur Verbesserung der Gleiteigenschaften zwischen Führungsrohr 14 und Instrumentengehäuse 10 trägt das proximale Ende des Führungsrohrs 14 einen an der Innenwand der Axialbohrung 12 geführten Gleitring 42, während das distale Ende der Axialbohrung 12 des Instrumentengehäuses 10 mit einer Stopfbuchse 44 aus Kunststoff für den Durchtritt des Führungsrohrs 14 versehen ist.

Das Instrumentengehäuse 10 ist in einem Drehlager 46 des Betätigungsschenkels 34 um die Längsachse der Axialbohrung 12 verdrehbar angeordnet. Das Verdrehen des Instrumentengehäuses 10 erfolgt über ein Wellenrad 48, das im Bereich zwischen den beiden Betätigungsschenkeln 34, 36 radial über das Instrumentengehäuse 10 übersteht und beim einhändigen Festhalten der Federzange 38 mit dem Daumen gedreht werden kann. Mit einer vorzugsweise als Kugelrast ausgebildeten Rastvorrichtung 50 im Bereich des Drehlagers 46 wird erreicht, daß das Instrumentengehäuse 10 in Winkelabständen von 90° an der Federzange 38 spürbar, aber trotzdem lösbar einrastet. Mit dem drehbaren Instrumentengehäuse soll der Operateur die Möglichkeit erhalten, die Branchen einhändig intraoperativ in ihrer Winkelstellung auszurichten.

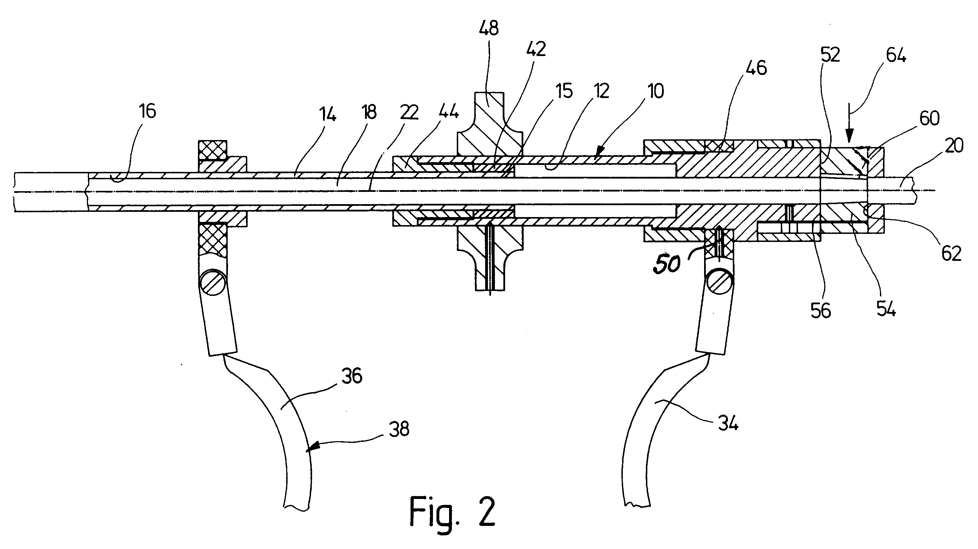

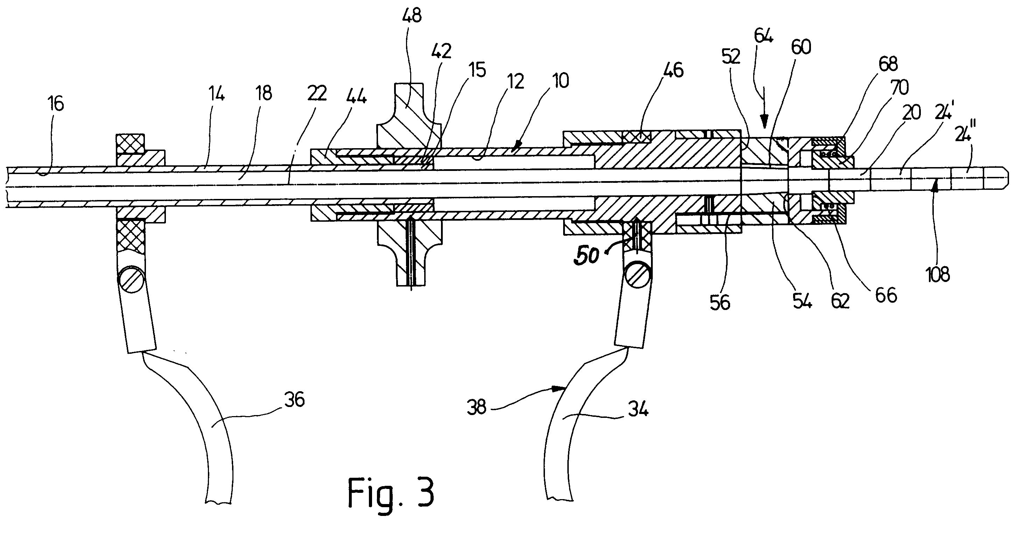

In der Nähe des proximalen Endes des Instrumentengehäuses ist in einer radialen Bohrung 52 ein Rastschieber 54 entgegen der Kraft einer am Instrumentengehäuse 10 festgelegten Blattfeder 56 begrenzt verschiebbar angeordnet. Der Rastschieber 54 weist eine zum proximalen Ende hin konvergierende konische Durchstecköffnung für das Elektrodenrohr 18 auf. Das Elektrodenrohr 18 ist seinerseits mit einer konischen Eindrehung 60 versehen, die durch eine zum distalen Ende weisende ringförmige Rastschulter 62 nach hinten begrenzt ist. Wie insbesondere aus Fig. 2 und 3 zu ersehen ist, gleitet das Elektrodenrohr 18 beim Einstecken in das Führungsrohr 14 und das Instrumentengehäuse 10 auf dem der Blattfeder 56 zugewandten Innenwandteil der exzentrisch angeordneten Durchstecköffnung unter Verbiegen der Blattfeder 56 auf, bis der Rastschieber 54 mit seinem proximalen Ende unter der Einwirkung der Blattfeder 56 in die konische Eindrehung 60 vor der Rastschulter 62 einrastet. Zum Entnehmen des Elektrodenrohrs 18 kann der Rastschieber 54 in Richtung des Pfeils 64 entgegen der Kraft der Blattfeder 56 eingedrückt und die Rastverbindung gelöst werden. Mit dem in Fig. 3 gezeigten Auswurfmechanismus, bestehend aus einem gegen eine Ringschulter 66 des Elektrodenrohrs 18 anliegenden, unter der Einwirkung einer Druckfeder 68 stehenden Auswurfkolben 70, wird das Elektrodenrohr 18 in der Ausraststellung aus dem Eingriffsbereich des Rastschiebers 54 zum distalen Ende hin ausgestoßen, so daß das Elektrodenrohr nach einmaligem Niederdrücken des Rastschiebers entnommen werden kann, ohne daß der Rastschieber versehentlich wieder einrasten kann. Die vorstehend beschriebenen Maßnahmen gewährleisten, daß das Elektrodenrohr 18 mit beliebiger Drehorientierung bezüglich seiner Längsachse 22 in das Führungsrohr 14 eingesteckt und eingerastet werden kann. Gleichzeitig wird damit in Kauf genommen, daß das Elektrodenrohr 18 aufgrund der rotationssymmetrischen Ausbildung bezüglich seiner Längsachse auch im eingerasteten Zustand gegenüber dem Gehäuse 10 verdreht werden kann. Zur Herstellung einer drehfesten Verbindung zwischen dem im Drehlager 46 verdrehbaren Instrumentengehäuse 10 und dem Elektrodenrohr 18 ist daher zusätzlich eine Drehkupplung erforderlich.

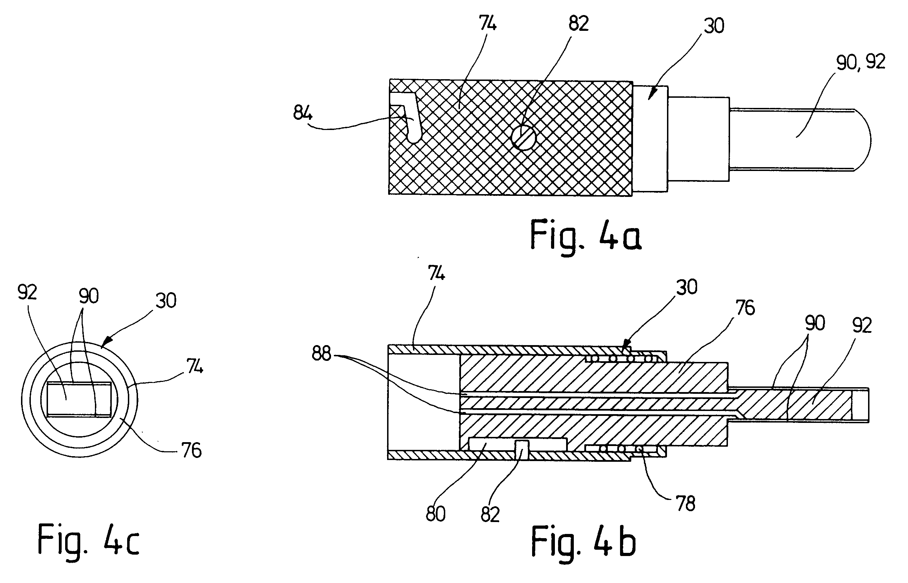

Zur Herstellung dieser Drehkupplung ist die in Fig. 4, 5 und 6 gezeigte Kupplungsvorrichtung 30 vorgesehen, die auf das proximale Ende des Instrumentengehäuses 10 und die Kontaktelemente 24 des Elektrodenrohrs 18 aufsteckbar ist. Die Kupplungsvorrichtung 30 besteht im wesentlichen aus einem Hülsenteil 74 zur Befestigung am Instrumentengehäuse 10 und einem Buchsenteil 76 zum Aufstecken auf die Kontaktelemente 24 des Elektrodenrohrs 18. Das Buchsenteil 76 ist im Hülsenteil 74 entgegen der Kraft einer Druckfeder 78 axial verschiebbar angeordnet. Das Buchsenteil weist zu diesem Zweck eine axial ausgerichtete Führungsnut 80 für den Eingriff eines als Schraubbolzen ausgebildeten Gleitsteins 82 auf. Der in die Führungsnut 80 eingreifende Gleitstein 82 bildet zugleich eine Verdrehsicherung zwischen Buchsenteil 76 und Hülsenteil 74. Das Hülsenteil 74 weist stirnseitig eine randoffene, abgebogene Bajonettkulisse 84 auf, in die unter Herstellung eines Bajonettverschlusses ein an der Oberfläche des Instrumentengehäuses 10 radial überstehender Bajonettzapfen 86 einführbar ist.

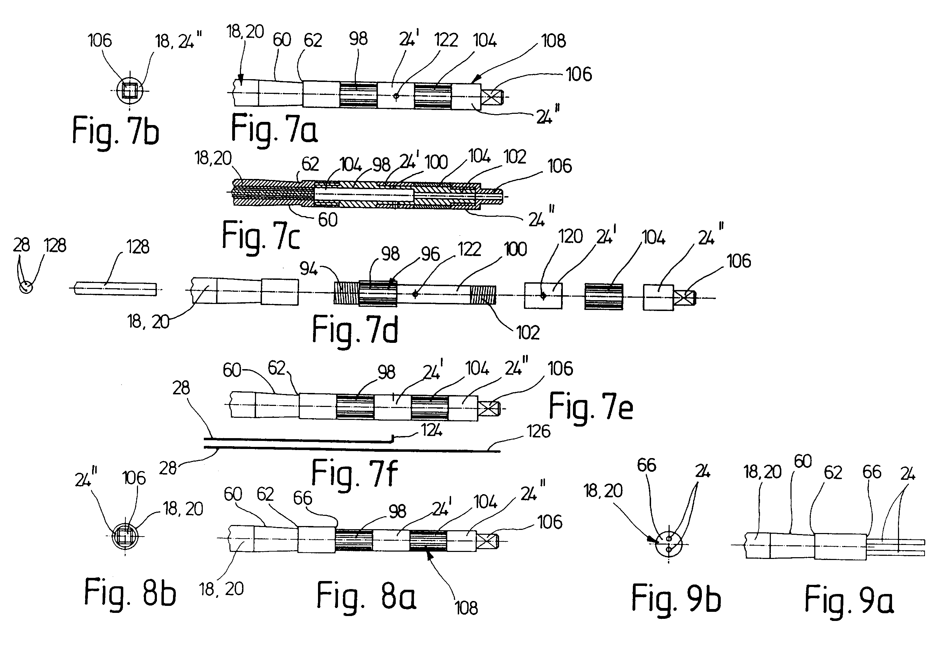

Die in Fig. 4a bis c gezeigte Kupplungsvorrichtung 30 ist für die in Fig. 9a und b gezeigten, axial über das proximale Elektrodenrohrende paarweise achsparallel überstehenden stiftförmigen Kontaktelemente 24 bestimmt. Das Buchsenteil 76 weist hierzu metallische Steckhülsen 88 zur Aufnahme der Kontaktstifte 24 auf, die elektrisch mit den beiden Polplatten 90 eines über das Buchsenteil 76 überstehenden Adpaterzapfens 92 verbunden sind.

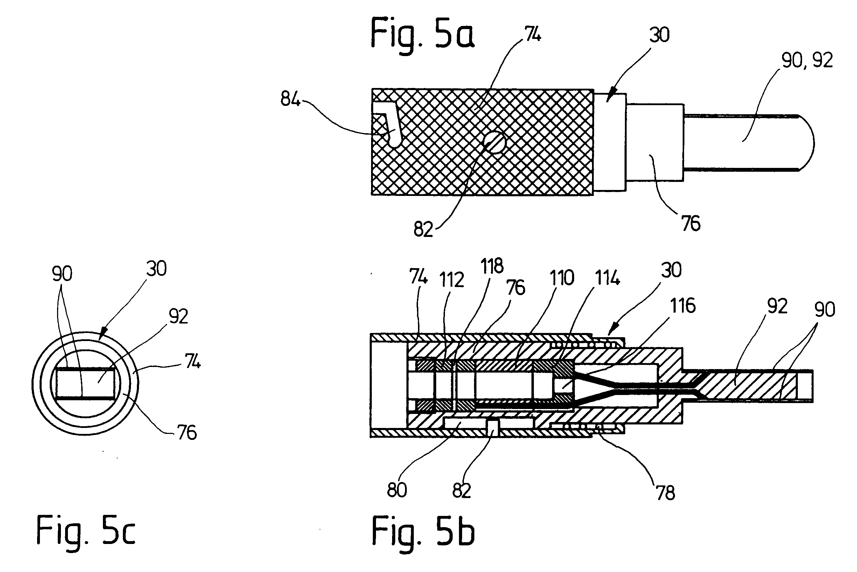

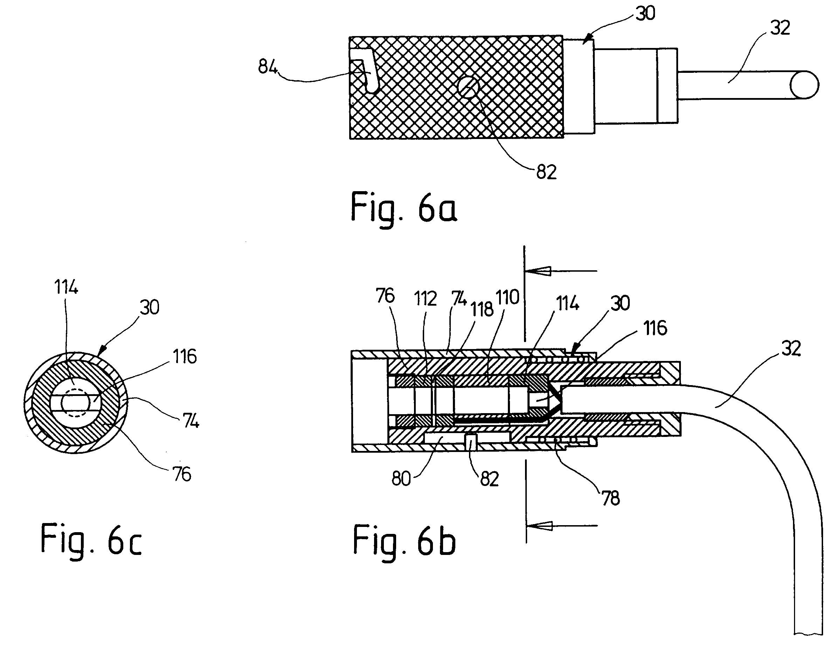

Die in Fig. 5a bis c, 6a bis c gezeigten Kupplungsvorrichtungen 30 sind zur Aufnahme der in Fig. 7a bis e sowie 8a und b gezeigten Segmentstecker bestimmt. Die ringförmigen Kontaktelemente 24 sind hier auf einer als Isolator ausgebildeten, mittels eines Gewindes 94 am proximalen Ende des Elektrodenrohrs 18 befestigten Rohrverlängerung 96 angeordnet. Die Rohrverlängerung 96 weist einen am Elektrodenrohr 18 fluchtend anschließenden Bund 98 sowie ein axial über den Bund überstehendes Rohrstück 100 mit kleinerem Durchmesser und einen axial überstehenden Gewindezapfen 102 auf. Auf das Rohrstück 100 sind vom freien Ende her ein das eine Kontaktelement bildender Metallring 24' und ein Isolatorring 104 aufgesteckt, die durch ein auf den Gewindezapfen 102 aufgedrehtes, das zweite Kontaktelement 24'' bildendes Abschlußstück in ihrer aufgesteckten Lage festgehalten werden. Das Abschlußstück 24'' weist einen Bund 106 sowie ein über den Bund überstehendes, vierkantiges Formstück 106 auf.

Die Drahtstücke 28 werden nacheinander in das auf diese Weise vorkonfektionierte Elektrodenrohr 18 eingesteckt. Zur Kontaktierung wird das eine Drahtstück 28 mit einem abgebogenen Ende 124 durch Radialbohrungen 120 und 122 im Rohrstück 100 und im Metallring 24' eingesteckt, während das gerade Drahtstück 28 mit seinem proximalen Ende 126 in eine Axialbohrung des metallischen Abschlußteils 24'' im Bereich des Formstücks 106 eingeführt wird. Sodann werden die Drahtstücke an ihren Enden vorzugsweise durch Laserschweißung mit dem zugehörigen Kontaktelement 24',24'' verbunden, wodurch gleichzeitig die betreffenden Bohrungen 120 abgedichtet werden.

Um eine ausreichende elektrische Durchschlagsicherheit zu erzielen, ist es zweckmäßig, wenn die Drahtstücke 28 als solche mit einer Isolierschicht versehen und zusätzlich durch einen doppellumigen isolierenden Schlauch 128 hindurchgeführt sind.

Der auf diese Weise gebildete zweipolige Segmentstecker 108 ist in das Buchsenteil 76 der in Fig. 5b und 6b gezeigten Kupplungsvorrichtung 30 unter Herstellung eines elektrischen Kontakts und einer drehfesten Verbindung einsteckbar. Das Buchsenteil 76 weist zu diesem Zweck zwei durch einen Isolatorring 110 voneinander getrennte Kontaktringe 112, 114 auf, von denen zumindest der Kontaktring 114 verdrehsicher im Buchsenteil 76 angeordnet ist. Der Kontaktring 114 weist zusätzlich eine Mehrkantöffnung 116 auf, die zur Herstellung einer formschlüssigen Verbindung mit dem Formstück 106 des Segmentsteckers 108 bestimmt ist. Der elektrische Kontakt im Bereich des Kontaktrings 114 wird unterstützt durch die Druckfeder 78, während der Kontaktring 112 zusätzlich eine Schlitzaussparung 118 zur Aufnahme einer kraftschlüssig gegen das betreffende Kontaktelement 24' anliegenden Kontaktfeder aufweist. Bei dem in Fig. 5a bis c gezeigten Ausführungsbeispiel sind die Kontaktringe 112,114 mit den Polplatten 90 eines Adapterzapfens 92 verbunden, während bei dem Ausführungsbeispiel nach Fig. 6a bis c ein zweipoliges HF-Kabel 32 an die Kontaktringe 112,114 angeschlossen ist. Das HF-Kabel 32 ist dabei über eine Zugentlastung im Buchsenteil 76 festgelegt.

Zusammenfassend ist folgendes festzustellen: Die Erfindung bezieht sich auf ein elektrochirurgisches Instrument zur bipolaren Koagulation oder Präparation von biologischen Geweben. Das Instrument weistzwei federnd ausgebildete, elektrisch voneiander isolierte Branchen 26 auf, die beim Verschieben eines Führungsrohrs 14 mit Hilfe eines Federgriffs 38 zangenartig unter Einklemmen des zu koagulierenden oder trennenden Gewebes gegeneinander bewegt werden. Durch Anlegen einer Hochfrequenzspannung fließt ein Hochfrequenzstrom über das eingeklemmte Gewebe, das bei ausreichend hohem Stromfluß erwärmt und koaguliert oder verdampft wird. Um das Elektrodenrohr mit seinen Branchen mit wenigen Handgriffen rasch und zuverlässig austauschen und auch während der Operation einhändig in seiner Drehlagenorientierung verändern zu können, wird gemäß der Erfindung vorgeschlagen, daß das Instrumentengehäuse 10 um seine Längsachse am einen Schenkel 34 des Federgriffs 38 drehbar gelagert ist, während das Elektrodenrohr 18 mit beliebiger Drehorientierung um seine Längsachse am Instrumentengehäuse 10 axial einrastbar und mit einer am Gehäuse 10 dreh- und verschiebbar verankerbaren, vorzugsweise einen Stromanschluß enthaltenden Kupplungsvorrichtung drehfest verbindbar ist.

## Patentansprüche

1. Elektrochirurgisches Instrument zur bipolaren Koagulation oder Präparation mit einem Gehäuse (10), mit einem in eine Axialbohrung (12) des Gehäuses (10) mit seinem proximalen Ende (15) eingreifenden und in dieser längsverschiebbar angeordneten Führungsrohr (14), mit einem durch das Führungsrohr (14) axial verschiebbar hindurchgreifenden, mit seinem proximalen Ende (20) durch das Führungsrohr (14) hindurch in die Axialbohrung (12) des Gehäuses (10) eingreifenden und in diesem in beliebiger Drehorientierung um seine Längsachse axial arretierbaren Elektrodenrohr (18) und mit mindestens einem sich durch das Innere des Elektrodenrohrs (18) erstreckenden und gegenüber diesem elektrisch isolierten Drahtstück (28), wobei am distalen Ende zwei über das Elektrodenrohr (18) und das Führungsrohr (14) überstehende, beim Verschieben des Führungsrohrs (14) radial zangenartig gegeneinander bewegbare, an ihren freien Enden metallische Elektroden (40) tragende, gegeneinander isolierte Branchen (26) und am proximalen Ende zwei mit den Elektroden (40) elektrisch verbundene, mit Hochfrequenz-Strom beaufschlagbare Kontaktelemente (24,24',24'') angeordnet sind, und wobei das Gehäuse (10) am einen Betätigungsschenkel (34) und das Führungsrohr (14) am anderen Betätigungsschenkel (36) einer vorzugsweise als Feder- oder Scherengriff ausgebildeten zweischenkeligen Betätigungsvorrichtung (38) angelenkt sind, **dadurch gekennzeichnet**, daß das Gehäuse (10) um die Achse seiner Axialbohrung (12) am einen Betätigungsschenkel (34) drehbar gelagert ist, und daß das Elektrodenrohr (18) mit beliebiger Drehorientierung um seine Längsachse (22) am Gehäuse (10) axial einrastbar und mit einer am Gehäuse (10) dreh- und verschiebefest verankerbaren Kupplungsvorrichtung (30) drehfest verbindbar ist.

2. Instrument nach Anspruch 1, **dadurch gekennzeichnet**, daß die Kupplungsvorrichtung (30) zugleich einen Stromanschluß für die Kontaktelemente (24,24',24'') enthält.

3. Instrument nach Anspruch 1 oder 2, **dadurch gekennzeichnet**, daß das Elektrodenrohr (18) als Schutzrohr ausgebildet ist, und daß in dem Elektrodenrohr (18) zwei gegeneinander elektrisch isolierte, an ihrem distalen Ende eine der Branchen (26) tragende Drahtstücke (28) angeordnet sind.

4. Instrument nach Anspruch 1 oder 2, **dadurch gekennzeichnet**, daß das Elektrodenrohr als Außenleiter und das Drahtstück als Innenleiter eines Koaxialleiters ausgebildet ist, wobei eine der Branchen am Elektrodenrohr und die andere Branche am Drahtstück angeordnet ist.

5. Instrument nach einem der Ansprüche 1 bis 4, **dadurch gekennzeichnet**, daß das Elektrodenrohr (18) eine isolierende Schutzhülle trägt.

6. Instrument nach einem der Ansprüche 1 bis 5, **dadurch gekennzeichnet**, daß die Kupplungsvorrichtung (30) eine auf einen radial überstehenden Bajonettzapfen (86) des Gehäuses (10) aufsteckbare Bajonettkulisse (84) aufweist.

7. Instrument nach einem der Ansprüche 1 bis 6, **dadurch gekennzeichnet**, daß die Kupplungsvorrichtung (30) ein Buchsenteil (76) zur Aufnahme der Kontaktelemente (24,24',24'') des Elektrodenrohrs (18) aufweist.

8. Instrument nach Anspruch 7, **dadurch gekennzeichnet**, daß die Kontaktelemente (24) als achsparallel über das proximale Elektrodenrohrende (20) überstehende Steckstifte ausgebildet sind, und daß die Kupplungsvorrichtung (30) zwei achsparallele Steckhülsen (88) zur formschlüssigen Aufnahme der Steckstifte (24) aufweist.

9. Instrument nach einem der Ansprüche 1 bis 7, **dadurch gekennzeichnet**, daß die Kontaktelemente (24', 24'') gegeneinander isoliert in axialem Abstand voneinander in der Nähe des proximale Elektrodenrohrendes (20) angeordnet sind und eine umlaufende, vorzugsweise zylindrische Kontaktfläche aufweisen, wobei das eine Kontaktelement (24'') ein das proximale Elektrodenrohrende (20) begrenzendes, vorzugsweise mehrkantiges oder seitlich abgeflachtes Formstück aufweist, das mit einem komplementären Formstück der Kupplungsvorrichtung (72) formschlüssig kuppelbar ist.

10. Instrument nach Anspruch 9, **gekennzeichnet durch** eine über das proximale Elektrodenrohrende (20) koaxial überstehende, vorzugsweise in dieses eingeschraubte, als Isolator ausgebildete Rohrverlängerung (96), die einen an das Elektrodenrohr (18) anschließenden Bund (98) etwa gleichen Durchmessers wie das Elektrodenrohr (18) und ein axial an den Bund (98) anschließendes Rohrstück (100) kleineren Durchmessers aufweist, auf dessen Rohrteil ein als erstes Kontaktelement ausgebildeter metallischer Kontaktring (24') und ein Isolatorring (104) aufsteckbar sind und an dessen freiem Ende ein den Kontaktring (24') und den Isolatorring (104) auf dem Rohrstück (100)festlegendes, das zweite Kontaktelement (24'') bildendes metallisches Abschlußteil mit umlaufender Kontaktfläche und rückwärtig überstehendem Formstück (106) befestigbar ist, wobei die Drahtstücke (28) mit je einem der Kontaktelemente (24',24'') verbunden, vorzugsweise verschweißt oder verlötet sind.

11. Instrument nach Anspruch 10, **dadurch** g**ekennzeichnet**, daß das Abschlußteil (24'') axial auf die Rohrverlängerung (96) aufgeschraubt ist.

12. Instrument nach Anspruch 10 oder 11, **dadurch gekennzeichnet**, daß die umlaufenden Kontaktflächen der Kontaktelemente (24',24'') und/oder die Umfangsflächen des Rohrverlängerungsbundes (98) und des Isolatorrings (104) den gleichen oder einen etwas kleineren Durchmesser wie das Elektrodenrohr(18)aufweisen.

13. Instrument nach einem der Ansprüche 9 bis 12, **dadurch gekennzeichnet**, daß der Kontaktring (24') und das Rohrstück (100) miteinander fluchtende Radialbohrungen (120, 122) zur Aufnahme des radial abgebogenen proximalen Endes (124) eines der Drahtstücke (28) aufweisen.

14. Instrument nach Anspruch 13, **dadurch gekennzeichnet**, daß das andere Drahtstück (28) mit seinem proximalen Ende (126) in eine Axialbohrung des metallischen Abschlußteils (24'') eingreift.

15. Instrument nach Anspruch 13 oder 14, **dadurch gekennzeichnet**, daß die Drahtstücke (28) an ihren proximalen Enden (124,126) vorzugsweise durch Laserschweißung mit dem zugehörigen Kontaktelement (24', 24'') verbunden sind.

16. Instrument nach einem der Ansprüche 1 bis 15, **dadurch gekennzeichnet**, daß das Elektrodenrohr (18) in der Nähe seines proximalen Endes eine zum distalen Ende weisende umlaufende koaxiale Rastschulter (62) aufweist.

17. Instrument nach Anspruch 16, **dadurch gekennzeichnet**, daß das Elektrodenrohr (18) eine an die Rastschulter (62) zur Stirnseite hin anschließende, vorzugsweise konische Eindrehung (60) aufweist.

18. Instrument nach Anspruch 16 oder 17, **gekennzeichnet durch** einen im Gehäuse (10), vorzugsweise in einer radialen Gehäusebohrung (52) radial verschiebbar angeordneten, eine Durchstecköffnung (58) für das Elektrodenrohr (18) aufweisenden Rastschieber (54), der unter der Einwirkung einer Rastfeder (56) im Bereich der Durchstecköffnung (58) hinter die Rastschulter (62) und/oder in die Eindrehung (60) des Elektrodenrohrs (18) lösbar einrastbar ist.

19. Instrument nach Anspruch 18, **dadurch gekennzeichnet**, daß die Durchstecköffnung (58) eine zum rückwärtigen Ende konvergierende konische Innenwand mit Übermaß gegenüber der konischen Eindrehung (60) des Elektrodenrohrs aufweist.

20. Instrument nach einem der Ansprüche 1 bis 19, **dadurch gekennzeichnet**, daß das Elektrodenrohr (18) eine zum proximalen Ende (20) weisende Auswurfschulter (66) aufweist, gegen die ein vorzugsweise im Gehäuse (10) begrenzt verschiebbar angeordnetes Auswurforgan (70) unter der Einwirkung einer Auswurffeder (68) andrückbar ist.

21. Instrument nach einem der Ansprüche 1 bis 20, **dadurch gekennzeichnet**, daß an dem Gehäuse (10), vorzugsweise an einem in dem Bereich zwischen den beiden Schenkeln (34,36) der Betätigungsvorrichtung (38) angeordneten Teil, ein zweckmäßig als Wellenrad ausgebildetes Betätigungsrad (48) radial überstehend angeordnet ist.

22. Instrument nach einem der Ansprüche 1 bis 21, **dadurch gekennzeichnet**, daß das Gehäuse (10) in Drehrichtung, vorzugsweise in Winkelabständen von jeweils 90° an der Betätigungsvorrichtung (38) lösbar einrastbar ist.

23. Instrument nach einem der Ansprüche 1 bis 22, **dadurch gekennzeichnet**, daß das Führungsrohr (14) mit einem endseitig aufgesetzten Gleit- und Anschlagring (42) aus Kunststoff in der Axialbohrung (12) des Gehäuses (10) begrenzt axial geführt ist.

24. Instrument nach einem der Ansprüche 1 bis 23, **dadurch gekennzeichnet**, daß die Axialbohrung (12) des vorzugsweise metallischen Gehäuses (10) durch eine zweckmäßig stirnseitig eingeschraubte, als stirnseitiger Anschlag ausgebildete Stopfbuchse (44) aus Kunststoff für den Durchtritt des vorzugsweise metallischen Führungsrohrs (14) begrenzt ist.

25. Instrument nach einem der Ansprüche 1 bis 24, **dadurch gekennzeichnet**, daß die Kupplungsvorrichtung (30) ein vorzugsweise mit einer Bajonettkulisse (84) zur Verankerung am Gehäuse (10) versehenes Hülsenteil (74) und ein drehfest gegenüber dem Hülsenteil (74) axial entgegen der Kraft einer Feder (78) verschiebbares Buchsenteil (76) aufweist.

26. Instrument nach Anspruch 25, **dadurch gekennzeichnet**, daß das Hülsenteil (74) einen in eine achsparallele Führungsnut (80) des Buchsenteils (76) eingreifenden, vorzugsweise als Schraubbolzen ausgebildeten Gleitstein (82) aufweist.

27. Instrument nach Anspruch 25 oder 26, **dadurch gekennzeichnet**, daß in dem Buchsenteil (76) zwei gegeneinander isoliert im Abstand voneinander angeordnete, miteinander fluchtende Buchsenringe (112, 114) drehfest angeordnet sind, von denen der eine, rückwärtig angeordnete Buchsenring (114) eine zum proximalen Formstück (106) des Elektrodenrohrs komplementäre Aussparung (116) aufweist.

28. Instrument nach einem der Ansprüche 1 bis 27, **dadurch gekennzeichnet**, daß die Elektroden (40) eine ballige, vorzugsweise halbkugelförmige oder halbzylinderförmige Gestalt aufweisen.

29. Instrument nach Anspruch 28, **dadurch gekennzeichnet**, daß die einander zugewandten Elektrodenflächen der beiden Elektroden eben sind.

30. Instrument nach einem der Ansprüche 1 bis 27, **dadurch gekennzeichnet**, daß die Elektroden (40) zueinander parallele, hakenförmige Abbiegungen aufweisen.

## Claims

1. An electro-surgical instrument for bipolar coagulation or dissection, comprising a casing (10), a guide tube (14) which is engaged with its proximal end (15) in an axial bore (12) of the casing (10) and which is adapted to longitudinally slide within the casing, comprising an electrode tube (18) which axially movably extends through the guide tube (14), the proximal end (20) of the electrode tube (18) protruding through the guide tube (14) into the axial bore (12) of the casing (10), and the electrode tube (18) being axially lockable within the guide tube (14) at any desired position of rotation about its longitudinal axis, and comprising at least one wire piece (28) which extends through the inside of the electrode tube (18) and which is electrically insulated with respect to the electrode tube (18), wherein two branches (26) which are insulated with respect to each other are positioned at the distal end, which branches (26) protrude over the electrode tube (18) and the guide tube (14), are radially movable toward each other in a clamp-like manner during the movement of the guide tube (14), and carry metallic electrodes (40) at their free ends, wherein two contact elements (24, 24', 24'') which are electrically connected to the electrodes (40) and are subjectable to a high frequency current are positioned at the proximal end, and wherein the casing (10) is connected to a first actuating leg (34) and the guide tube (14) is connected to a second actuating leg (36) of two-legged actuating means (38) which are preferably designed to be spring- or scissor-handles, **characterized in that** the casing (10) is borne at the first actuating leg (36) in a manner permitting rotation about the axis of its axial bore (12), and that the electrode tube (18) is axially lockable at the casing (10) at any desired position of rotation about its longitudinal axis and unrotatably connectable to a coupling device (30) which is unrotatably and unmovably anchorable at the casing (10).

2. The instrument of claim 1, **characterized in that** the coupling device (30) also comprises an electrical connection for the contact elements (24, 24', 24'').

3. The instrument of claim 1 or 2, **characterized in that** the electrode tube (18) is formed to be a protecting tube, and that two wire pieces (28) which are electrically insulated with respect to each other and each of which carries one of the branches (26) at its distal end are positioned within the electrode tube (18).

4. The instrument of claim 1 or 2, **characterized in that** the electrode tube is formed to be the outer conductor and the wire piece is formed to be the inner conductor of a coaxial conductor, wherein one of the branches is positioned on the electrode tube and the other branch is positioned on the wire piece.

5. The instrument of one of claims 1 to 4, **characterized in that** the electrode tube (18) has an insulating protective outer cover.

6. The instrument of one of claims 1 to 5, **characterized in that** the coupling device (30) has a bayonet slide (84) which can be coupled to to a radially protruding bayonet base (86) on the casing (10).

7. The instrument of one of claims 1 to 6, **characterized in that** the coupling device (30) has a socket (76) for accepting the contact elements (24, 24', 24'') of the electrode tube (18).

8. The instrument of claim 7, **characterized in that** the contact elements (24) are formed to be pins which protrude in an axially parallel direction over the proximal electrode tube end (20), and that the coupling device (30) has two axially parallel sockets (88) for accepting the pins (24).

9. The instrument of one of claims 1 to 7, **characterized in that** the contact elements (24, 24', 24'') are positioned spaced and insulated with respect to each other in the vicinity of the proximal electrode tube end (20) and that they have a circumferential, preferably cylindrical, contact area, wherein the one contact element (24'') has a formed part which delimits the proximal electrode tube end (20), which formed part is preferably multi-sided or laterally flattened and which is positively coupleable to a complimentary formed part of the coupling device (72).

10. The instrument of claim 9, **characterized by** a tube extension (96) which is formed to be an insulator and which coaxially protrudes over the proximal electrode tube end (20), preferably being screwed into this, the tube extension (96) having a collar (98) of approximately the same diameter as the electrode tube (18) connected to the electrode tube (18) and a tube piece (100) of a smaller diameter axially connected to the collar (98), onto the tubular part of which are mountable a contact ring (24') and an insulator ring (104) and to the free end of which is connectable a metallic end piece which forms the second contact element (24'') and fixes the contact ring (24') and the insulator ring (104) to the tube piece (100) and which has a circumferential contact area and a rearwardly protruding formed part (106), wherein the wire pieces (28) each are connected to one of the contact elements (24', 24''), preferably by means of welding or soldering.

11. The instrument of claim 10, **characterized in that** the end piece (24'') is axially screwed onto the tube extension (96).

12. The instrument of claim 10 or 11, **characterized in that** the circumferential contact areas of the contact elements (24', 24") and/or the circumferential areas of the tube extension collar (98) and of the insulator ring (104) have the same or a slightly smaller diameter as the electrode tube (18).

13. The instrument of one of claims 9 to 12, **characterized in that** the contact ring (24') and the tube part (100) have aligned radial bores (120, 122) for accepting the radially bent-off proximal end (124) of one of the wire pieces (28).

14. The instrument of claim 13, **characterized in that** the other wire piece (28) extends with its proximal end (126) into an axial bore of the metallic end piece (24'').

15. The instrument of claim 13 or 14, **characterized in that** the wire pieces (28) are connected with their proximal end (124, 126) to the corresponding contact element (24', 24"), preferably by means of laser welding.

16. The instrument of one of claims 1 to 15, **characterized in that** the electrode tube (18) has in the vicinity of its proximal end a circumferential coaxial locking shoulder (62) which is directed toward the distal end.

17. The instrument of claim 16, **characterized in that** the electrode tube (18) has a preferably conical recess (60) adjacent to the face side of the locking shoulder (62).

18. The instrument of claim 16 or 17, **characterized by** a notched slider (54) which is positioned in the casing (10), preferably radially movably in a radial casing bore (52), and which has a penetration opening (58) for the electrode tube (18), the notched slider (54) being removably lockable behind the locking shoulder (62) and/or the recess (60) under the action of a locking spring (56) in the region of the penetration opening (58).

19. The instrument of claim 18, **characterized in that** the penetration opening (58) has an inner wall which is conically tapered toward the rear end and which is oversized with respect to the conical recess (60) of the electrode tube.

20. The instrument of one of claims 1 to 19, **characterized in that** the electrode tube (18) has an ejection shoulder (66) directed toward the proximal end (20), against which an ejection member (70), which is preferably adapted to be limitedly movable in the casing (10), can be pressed under the action of an ejection spring (68).

21. The instrument of one of claims 1 to 20, **characterized in that** an actuating wheel (48), preferably a shaft wheel positioned on a part in the region between the two legs (34, 36) of the actuating mechanism (38), is positioned radially protruding on the casing (10).

22. The instrument of one of claims 1 to 21, **characterized in that** the casing (10) is removably lockable on the actuating mechanism (38) in the direction of rotation, preferably at angular spacings of 90°.

23. The instrument of one of claims 1 to 22, **characterized in that** the guide tube (14) is limitedly axially guided in the axial bore (12) of the casing (10) by means of a plastic slide- and stop-ring (42) which is positioned on the end side of the guide tube.

24. The instrument of one of claims 1 to 23, **characterized in that** the axial bore (12) of the preferably metallic casing (10) is delimited by a plastic packing gland (44) formed to be a face-end stop through which the preferably metallic guide tube (14) passes, the packing gland being screwed into the front face side of the casing.

25. The instrument of one of claims 1 to 24, **characterized in that** the coupling device (30) has a sleeve part (74) which preferably has a bayonet slide (84) to fix it to the casing (10) and a bushing part (76) which is axially movable unrotatably with respect to the sleeve part (74) against the force of a spring (78).

26. The instrument of claim 25, **characterized in that** the sleeve part (74) has a slide block (82) which is engaged in an axially parallel guide groove (80) of the bushing part (76) and which is preferably formed to be a threaded bolt.

27. The instrument of claim 25 or 26, **characterized in that** two aligned sleeve rings (112, 114) are positioned spaced and insulated with respect to each other nonrotatably within the bushing part (76), of which sleeve rings the rearwardly positioned one has a recess (116) which is complementary to the proximal formed part (106) of the electrode tube.

28. The instrument of one of claims 1 to 27, **characterized in that** the electrodes (40) have a spherical, preferably semi-spherical or semi-cylindrical shape.

29. The instrument of claim 28, **characterized in that** the surfaces of the electrodes which face each other are plane.

30. The instrument of one of claims 1 to 27, **characterized in that** the electrodes (40) have bent-off sections which are parallel to each other and have a hook-like shape.

## Revendications

1. Instrument électro-chirurgical pour la coagulation ou la préparation bipolaire de tissus, comprenant un boitier (10), un tube de guidage (14) s'engageant avec son extrémité proximale (15) dans un alésage axial (12) du boîtier (10) dans lequel il peut être déplacé dans le sens longitudinal, un tube d'électrode (18) traversant le tube de guidage (14) de manière mobile dans le sens axial dont l'extrémité proximale (20) s'engage, au travers du tube de guidage (14), dans l'alésage axial (12) du boîtier (10) et qui peut être bloqué axialement dans celui-ci, autour de son axe longitudinal, dans une position de rotation quelconque, et au moins une section de fil métallique (28) s'étendant au travers de l'intérieur du tube d'électrode (18) et électriquement isolée par rapport à celui-ci, à l'extrémité distale étant disposées deux branches (26) isolées l'une par rapport à l'autre, dépassant du tube d'électrode (18) et du tube de guidage (14) et déplacable radialement l'une par rapport à l'autre, à la manière d'une pince, lors du déplacement du tube de guidage (14), dont les extrémités libres portent des électrodes métalliques (40), alors qu'à l'extrémité proximale sont disposés deux éléments de contact (24, 24', 24'') électriquement reliés aux électrodes (40) et pouvant être alimentés en courant de haute fréquence, et le boîtier (10) étant articulés sur l'une des branches de manoeuvre (34) et le tube de guidage (14), sur l'autre branche de manoeuvre (36) d'un dispositif de manoeuvre (38) à deux branches conformé de préférence en pince ou poignée de cisailles, **carac-térisé en ce** que le boîtier (10) est monté sur l'une des branches de manoeuvre (34) de manière à pouvoir tourner autour de l'axe de son alésage axial (12), et que le tube d'électrode (18) peut s'enclencher axialement, dans une position angulaire quelconque autour de son axe longitudinal (22) sur le boîtier (10) et être solidarisé en rotation avec un dispositif d'accouplement (30) pouvant être ancré de manière rigide en rotation et en translation sur le boîtier (10).

2. Instrument selon la revendication 1, caractérisé en ce que le dispositif d'accouplement (30) contient en même temps un branchement électrique pour les éléments de contact (24, 24', 24'').

3. Instrument selon la revendication 1 ou 2, caractérisé en ce que le tube d'électrode (18) est conformé en tube protecteur et que dans le tube d'électrode (18) sont disposées deux sections de fil métallique (28) électriquement isolées l'une de l'autre et portant à leur extrémité distale l'une des branches (26).

4. Instrument selon la revendication 1 ou 2, caractérisé en ce que le tube d'électrode est conformé en conducteur extérieur, et la section de fil métallique en conducteur intérieur d'un conducteur coaxial, l'une des branches étant placée sur le tube d'électrode et l'autre branche étant disposée sur la section de fil métallique.

5. Instrument selon l'une des revendications 1 à 4, caractérisé en ce que le tube d'électrode (18) porte une gaine protectrice isolante.

6. Instrument selon l'une des revendications 1 à 5, caractérisé en ce que le dispositif d'accouplement (30) comporte une coulisse à baïonnette (84) emboîtable sur un pivot de baïonnette (86) du boîtier (10) en saillie radiale.

7. Instrument selon l'une des revendications 1 à 6, caractérisé en ce que le dispositif d'accouplement (30) comporte un élément de douille (76) pour la réception des éléments de contact (24, 24', 24'') du tube d'électrode (18).

8. Instrument selon la revendication 7, caractérisé en ce que les éléments de contact (24) sont réalisés sous la forme de fiches de contact dépassant parallèlement à l'axe de l'extrémité proximale (20) du tube d'électrode, et que le dispositif d'accouplement (30) comporte deux alvéoles (88) parallèles à l'axe pour la réception à engagement positif des fiches de contact (24).

9. Instrument selon l'une des revendications 1 à 7, caractérisé en ce que les éléments de contact (24', 24''), isolés l'un de l'autre, sont disposés à distance axiale l'un de l'autre à proximité de l'extrémité proximale (20) du tube d'électrode et présentent une surface de contact circulaire, de préférence cylindrique, l'un des éléments de contact (24'') présentant un élément profilé, de préférence polygonal ou latéralement aplati, qui limite l'extrémité proximale (20) du tube d'électrode et peut être couplé à engagement positif avec un élément profilé complémentaire du dispositif d' accouplement (72).

10. Instrument selon la revendication 9, caractérisé en ce qu'il comprend une rallonge de tube (96) conformée en isolateur qui dépasse coaxialement de l'extrémité proximale (20) du tube d'électrode et comporte un collet (98) raccordé au tube d'électrode (18) et présentant sensiblement le même diamètre que le tube d'électrode (18), et un segment de tube (100) d'un diamètre plus faible qui se raccorde axialement audit collet (98), une bague de contact métallique (24') formant le premier élément de contact et une bague isolante (104) pouvant être emboîtées sur ledit segment de tube à l'extrémité libre duquel peut être fixé un élément terminal métallique qui bloque la bague de contact (24') et la bague isolante (104) sur le segment de tube (100) et forme le second élément de contact (24''), avec une surface de contact circulaire et une partie profilée (106) en saillie vers l'arrière, les sections de fil métallique (28) étant chacune reliées, de préférence soudées ou brasées, à l'un des éléments de contact (24', 24'').

11. Instrument selon la revendication 10, caractérisé en ce que l'élément terminal (24'') est vissé axialement sur la rallonge de tube (96).

12. Instrument selon la revendication 10 ou 11, caractérisé en ce que les surfaces de contact circulaires des éléments de contact (24', 24'') et/ou les surfaces périphériques du collet (98) de la rallonge de tube et de la bague isolante (104) présentent un diamètre égal ou légèrement inférieur à celui du tube d'électrode (18).

13. Instrument selon l'une des revendications 9 à 12, caractérisé en ce que la bague de contact (24') et le segment de tube (100) présentent des alésages radiaux (120, 122) alignés pour la réception de l'extrémité proximale (124) coudée dans le sens radial de l'une des sections de fil métallique (28).

14. Instrument selon la revendication 13, caractérisé en ce que l'autre section de fil métallique (28) s'engage avec son extrémité proximale (126) dans un alésage axial de l'élément terminal métallique (24'').

15. Instrument selon la revendication 13 ou 14, caractérisé en ce que les sections de fil métallique (28) sont reliées à leur extrémité proximale (124, 126), par soudage à laser, à l'élément de contact (24', 24'') associé.

16. Instrument selon l'une des revendications 1 à 15, caractérisé en ce que le tube d'électrode (18) présente, à proximité de son extrémité proximale, un épaulement d'arrêt (62) circulaire coaxial dirigé vers l'extrémité distale.

17. Instrument selon la revendication 16, caractérisé en ce que le tube d'électrode (18) comporte une gorge tournée (60) de préférence conique qui fait suite à l'épaulement d'arrêt (62) en direction de la face frontale.

18. Instrument selon la revendication 16 ou 17, caractérisé en ce qu'il comprend un coulisseau d'arrêt (54) disposé dans le boîtier (10), de préférence de manière à pouvoir être déplacé radialement dans un alésage radial (52) du boîtier, et comportant une ouverture de passage (58) pour le tube d'électrode (18), lequel coulisseau d'arrêt peut s'enclencher de manière amovible, sous l'action d'un ressort à cran d'arrêt (56), dans la région de l'ouverture de passage (58), derrière l'épaulement d'arrêt (62 et/ou dans la gorge tournée (60) du tube d'électrode (18).

19. Instrument selon la revendication 18, caractérisé en ce que l'ouverture de passage (58) présente une paroi intérieure conique convergeant vers l'extrémité postérieure, avec une surmesure par rapport à la gorge conique tournée (60) du tube d'électrode.

20. Instrument selon l'une des revendications 1 à 19, caractérisé en ce que le tube d'électrode (18) comporte un épaulement d'éjection (66) dirigé vers l'extrémité proximale (20), contre lequel peut être appliqué, sous l'action d'un ressort d'éjection (68), un organe d'éjection (70) disposé dans le boîtier (10) de façon à pouvoir effectuer un déplacement limité.

21. Instrument selon l'une des revendications 1 à 20, caractérisé en ce que sur le boitier (10), de préférence sur un élément placé dans la région entre les deux branches (34, 36) du dispositif de manoeuvre (38), est montée en saillie radiale une roue de manoeuvre (48) avantageusement conformée en roue ondulée.

22. Instrument selon l'une des revendications 1 à 21, caractérisé en ce que le boîtier (10) peut s'enclencher de manière amovible dans la direction de rotation, de préférence à des intervalles angulaires de respectivement 90°, sur le dispositif de manoeuvre (38).

23. Instrument selon l'une des revendications 1 à 22, caractérisé en ce que le tube de guidage (14) est guidé axialement de manière limitée, avec une bague de glissement et d'arrêt (42) en matière plastique, dans l'alésage axial (12) du boîtier (10).

24. Instrument selon l'une des revendications 1 à 23, caractérisé en ce que l'alésage axial (12) du boitier (10), de préférence métallique, est délimité par un presse-étoupe (44) en matière plastique, avantageusement vissé du côté frontal et réalisé sous la forme d'une butée frontale, pour le passage du tube de guidage (14), de préférence métallique.

25. Instrument selon l'une des revendications 1 à 24, caractérisé en ce que le dispositif d'accouplement (30) comprend un élément de manchon (74) avantageusement muni d'une coulisse à baïonnette (84) pour l'ancrage sur le boîtier (10), et un élément de douille (76) solidaire en rotation, axialement déplaçable par rapport à l'élément de manchon (74) contre la force d'un ressort (78).

26. Instrument selon la revendication 25, caractérisé en ce que l'élément de manchon (74) comporte un coulisseau (82) conformé de préférence en boulon fileté qui s'engage dans une rainure de guidage (80) de l'élément de douille (76) orientée parallèlement à l'axe.

27. Instrument selon la revendication 25 ou 26, caractérisé en ce que dans l'élément de douille (76) sont disposées de manière solidaire en rotation, deux bagues de douille (112, 114) alignées, isolées l'une de l'autre et disposées à distance l'une de l'autre, dont la bague de douille (114) placée à l'arrière présente un évidement (116) complémentaire de l'élément profilé proximal (106) du tube d'électrode.

28. Instrument selon l'une des revendications 1 à 27, caractérisé en ce que les électrodes (40) présentent une forme bombée, de préférence hémisphérique ou semi-cylindrique.

29. Instrument selon la revendication 28, caractérisé en ce que les surfaces des deux électrodes tournées l'une vers l'autre sont planes.

30. Instrument selon l'une des revendications 1 à 27, caractérisé en ce que les électrodes (40) présentent des coudes en forme de crochets parallèles.
